(19) Europäisches Patentamt
European Patent Office
Office européen des brevets

(11) **EP 3 438 687 B1**

(12) **EUROPÄISCHE PATENTSCHRIFT**

(45) Veröffentlichungstag und Bekanntmachung des Hinweises auf die Patenterteilung:
**15.11.2023 Patentblatt 2023/46**

(21) Anmeldenummer: **18185494.4**

(22) Anmeldetag: **25.07.2018**

(51) Internationale Patentklassifikation (IPC):
**G01R 33/385** (2006.01)

(52) Gemeinsame Patentklassifikation (CPC):
**G01R 33/3852;** G01R 33/56572

(54) **ENDSTUFE ZUR ERZEUGUNG VARIABLER, RECHTECKFÖRMIGER STRÖME IN EINER INDUKTIVEN LAST OHNE HOCHSPANNUNGSVERSORGUNG**

OUTPUT STAGE FOR GENERATING VARIABLE, RECTANGULAR CURRENTS IN AN INDUCTIVE LOAD WITHOUT HIGH VOLTAGE SUPPLY

ÉTAGE FINAL DE PRODUCTION DE COURANTS VARIABLES, RECTANGULAIRES DANS UNE CHARGE INDUCTIVE SANS ALIMENTATION HAUTE TENSION

(84) Benannte Vertragsstaaten:
**AL AT BE BG CH CY CZ DK EE ES FI FR GB GR HR HU IE IS IT LI LT LU LV MC MK MT NL NO PL PT RO RS SE SI SK SM TR**

(30) Priorität: **31.07.2017 DE 102017213197**

(43) Veröffentlichungstag der Anmeldung:
**06.02.2019 Patentblatt 2019/06**

(73) Patentinhaber: **Bruker BioSpin GmbH
76287 Rheinstetten (DE)**

(72) Erfinder: **Maixner, Michael
76467 Bietigheim (DE)**

(74) Vertreter: **Kohler Schmid Möbus Patentanwälte
Partnerschaftsgesellschaft mbB
Gropiusplatz 10
70563 Stuttgart (DE)**

(56) Entgegenhaltungen:
**US-A- 4 961 054     US-A- 5 311 136**

- **SIQI LI ET AL: "Digital controlled MOSFET gradient amplifier for magnetic resonance imaging", ELECTRICAL MACHINES AND SYSTEMS (ICEMS), 2011 INTERNATIONAL CONFERENCE ON, IEEE, 20. August 2011 (2011-08-20), Seiten 1-6, XP032020715, DOI: 10.1109/ICEMS.2011.6073953 ISBN: 978-1-4577-1044-5**

EP 3 438 687 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft eine Magnetresonanzapparatur mit einem Hauptfeldmagneten zur Erzeugung eines homogenen Magnetfelds in einem Messvolumen, mit einer Gradientenanordnung zum Erzeugen von magnetischen Feldgradienten im Messvolumen, welche mindestens eine Gradientenspule mit einem Innenwiderstand umfasst, sowie mit einer elektrischen Schaltung zur Erzeugung variabler, impulsförmiger Ströme durch die Gradientenanordnung, welche ein erstes Schaltelement in Serie mit einem ersten Widerstand umfasst, mit dem ein Strom nur durch eine erste Hilfsspule ein- oder ausgeschaltet werden kann, und wobei die elektrische Schaltung ein zweites Schaltelement umfasst, mit welchem ein serieller Stromfluss durch die Gradientenspule und die erste Hilfsspule geschaltet werden kann.

Eine solche Magnetanordnung ist bekannt aus der DE 33 36 286 A1 (=Referenz [1]).

Hintergrund der Erfindung

Die vorliegende Erfindung betrifft allgemein den Bereich der Magnetresonanz (MR) mit geeigneten Magnetsystemen, die zur Erzeugung von homogenen Magnetfeldern für MR-Messungen bestimmt sind. Die Anwendbarkeit der Erfindung ist jedoch nicht auf dieses Gebiet beschränkt.

Sowohl im Bereich der Kernresonanzspektroskopie (NMR) als auch bei der bildgebenden Anwendung (MRI) wird in einem zu definierenden Probenvolumen ein sehr homogenes und zeitlich konstantes Magnetfeld benötigt, welches mit resistiven oder supraleitenden Spulen oder einer geeigneten Permanentmagnetanordnung erzeugt werden kann. Bildgebende Magnetresonanz(=MRI)-Verfahren werden vielfältig genutzt, um Bildinformationen von Strukturen zu erhalten. Dabei ist es möglich, Bildinformationen auch aus dem Inneren einer Struktur zu erhalten, ohne die Struktur zu beschädigen. Beispielsweise werden in klinischen Anwendungen Innenansichten der Körperteile von Menschen und Tieren mittels MRI-Verfahren abgebildet. Bei den heute verwendeten MRI-Verfahren wird den Präzessionsbewegungen der Kernspins durch zeitlich variierte Überlagerungen von zusätzlichen ortsabhängigen Magnetfeldern für alle drei Raumrichtungen eine räumliche Kodierung, im Allgemeinen als Ortskodierung bezeichnet, aufgeprägt. Diese Zusatzmagnetfelder weisen üblicherweise innerhalb des Untersuchungsobjekts im wesentlichen konstante Gradienten der z-Komponente in den Raumrichtungen x, y und z auf und werden von einer als Gradientensystem bezeichneten Spulenanordnung, die für die Raumrichtungen jeweils von einem sog. Gradientenkanal angesteuert werden, erzeugt.

Das Erzeugen eines Gradienten wird dadurch bewerkstelligt, dass durch eine Gradientenspule, welche das statische Magnetfeld an dem Untersuchungsobjekt verändert, ein impulsförmiger Strom geschickt wird. Wie etwa in der US 2006/0152222 A1 (=Referenz [2]) beschrieben, wird oft eine erhöhte Spannung, bis einige 100V, an die Gradientenspule angelegt, wodurch der Strom schneller ansteigt, um dann, wenn die gewünschte Stromstärke erreicht ist, auf eine niedrigere Spannung umzuschalten, um die Verlustleistung im Gradientenverstärker zu reduzieren. Für den ersten Fall wird aber, wenn auch nur für kurze Zeit eine hohe Spannung benötigt, die in der Schaltung des Gradientenverstärkers immer vorgehalten wird.

Definitionen

Als **Stromquelle** bezeichnet man in der Schaltungstheorie und Netzwerkanalyse der Elektrotechnik einen aktiven Zweipol, der an seinen Anschlusspunkten einen elektrischen Strom liefert. Als wesentliche Eigenschaft hängt dieser Strom nur gering, oder bei dem Modell der idealen Stromquelle im Rahmen der Schaltungsanalyse gar nicht, von der elektrischen Spannung an seinen Anschlusspunkten ab.

Als **Spannungsquelle** bezeichnet man in der Schaltungstheorie der Elektrotechnik einen aktiven Zweipol, der zwischen seinen Anschlusspunkten eine elektrische Spannung liefert. Als wesentliche Eigenschaft hängt diese Spannung nur gering, oder bei dem Modell der idealen Spannungsquelle im Rahmen der Netzwerkanalyse gar nicht, von dem elektrischen Strom ab, welcher der Quelle entnommen wird.

Die oben zitierte US 2006/0152222 A1 offenbart eine Gradientenschaltung mit einer Verstärkerschaltung der herkömmlichen Funktionsweise, wobei der Gradientenverstärker eine Vielzahl von Schaltstromreglern umfasst, die elektrisch in Reihe geschaltet sind. Eine bipolare Schaltung empfängt Leistung von der verbundenen Reihenschaltungs-Leistungsregler und liefert die Leistung einer ausgewählten Polarität an die Gradientenspule.

Eine Alternative zur Anordnung nach der US 2006/0152222 A1 bietet die Schaltung aus der eingangs zitierten DE 33 36 286 A1. Allerdings ist die dort verwendete Stromquelle, die, nach der Definition einer Stromquelle, einen hohen Ausgangswiderstand hat und einen konstanten Strom liefert, als Speisung nicht dafür geeignet, einen bestmöglich rechteckig geformten Stromanstieg in der Gradientenspule zu erzeugen. Bei der beschriebene Schaltung wird zunächst eine Hilfsspule von dem Strom aus der Stromquelle durchflossen, wodurch sich dort ein Magnetfeld aufbaut. In dem Moment, wenn der Gradientenstrom beginnen soll, wird dieser eingeschaltet und der Strom durch die Hilfsspule abgeschaltet. Es entsteht für kurze Zeit eine Hochspannung an der Hilfsspule. Durch die Verwendung der Stromquelle als Stromlieferant kann aber die in der Hilfsspule gespeicherte Energie nicht in die Gradientenspule fließen, weil der Ausgangswiderstand der Stromquelle hoch ist. Eine Beladung der Gradientenspule mittels einer Hilfsinduktivität wird in der US 2006/0152222 A1 nicht beschrieben.

Eine weitere Alternative bietet die Schaltung nach US-A 4,961,054 (=Referenz [3]). Hier kommt der gewünschte Gradientenstrom aus einem Verstärker. Da offenbar dieser Strom konstant gehalten wird, ist eine Hilfsspule mit einer 5 bis 20 mal höheren Induktivität nötig, als sie die Gradientenspule aufweist. Mit dieser große Induktivität dauert es sehr lange, bis der richtige Stromfluss erreicht ist, oder der die Schaltung speisende Verstärker braucht eine sehr große maximale Ausgangsspannung. So wird im Messsystem entweder eine sehr hohe Versorgungsspannung für den Verstärker benötigt, oder die Wartezeit zwischen zwei Gradientenimpulsen muss relativ lang sein. Auch beim Vorhandensein der vorgenannten Bedingungen ist es so nicht möglich, einen optimal impulsförmigen Stromanstieg in der Gradientenspule zu erzeugen. Dafür müsste die Hilfsspule eine unendlich große Induktivität haben, was eine unendliche Wartezeit ergäbe, oder der Verstärker müsste eine unendlich hohe maximale Ausgangsspannung haben. Beides ist in der Praxis nicht möglich.

Des Weiteren beschreibt die US-A 5,270,657 (=Referenz [4]) ein Gradientenverstärkersystem, in dem DC-Stromversorgungen in Tandem mit herkömmlichen linearen Gradientenverstärkern angeschlossen werden, um die effektive Gradientenenergie zu den Gradientenspulen zu steigern. Eine Beladung der Gradientenspule mittels einer Hilfsinduktivität ist auch hier nicht beschrieben.

Die US-A 2,941,125 (=Referenz [5]) offenbart eine Schaltung, um Ströme mit möglichst rechteckigem Profil in einer Induktivität zu erzeugen, um eine Mikrowellenvorrichtung zu schalten.

Aus US-A 5,311,136 (=Referenz [6]) schließlich ist eine Magnetresonanzapparatur mit einem Hauptfeldmagneten zur Erzeugung eines homogenen Magnetfelds in einem Messvolumen bekannt, mit einer Gradientenanordnung zum Erzeugen von magnetischen Feldgradienten im Messvolumen, welche mindestens eine Gradientenspule mit einem Innenwiderstand umfasst, sowie mit einer elektrischen Schaltung in Form einer Voll- oder H-Brücke zur Erzeugung variabler, impulsförmiger Ströme durch die Gradientenanordnung, welche elektrische Schaltung mindestens eine Spannungsquelle umfasst, mit der ein definierter Spannungsabfall über dem Innenwiderstand der Gradientenspule eingestellt werden kann, wobei eine Hilfsspule in der elektrischen Schaltung zwischen der Spannungsquelle und der Gradientenspule in Serie mit der Gradientenspule angeordnet ist, dass in der elektrischen Schaltung ein erster Schaltungsteil und ein zweiter Schaltungsteil vorhanden ist, wobei der zweite Schaltungsteil es ermöglicht, die zum ersten Schaltungsteil entgegengesetzte Stromrichtung in der Gradientenspule zu erzeugen.

## Aufgabe der Erfindung

Der vorliegenden Erfindung liegt demgegenüber die Aufgabe zugrunde, mit einfachen technischen Maßnahmen eine Magnetresonanzapparatur der eingangs definierten Art für eine MR-Vorrichtung bereitzustellen, bei welcher die elektrische Schaltung zur Erzeugung variabler, impulsförmiger Gradientenströme einen möglichst genau definierten Magnetfeldgradienten in einem Untersuchungsobjekt bei der magnetischen Kernresonanz zu erzeugen kann, wobei die Gradientenspule möglichst schnell auf die Zielstromstärke gebracht werden soll, wobei aber die Notwendigkeit einer hohen Versorgungsspannung umgangen wird. Damit sollen möglichst präzise Rechteck-Strompulse bei gleichzeitig kurzer Ladezeit der Hilfsinduktivität erzeugt werden.

## Kurze Beschreibung der Erfindung

Diese Aufgabe wird durch die vorliegende Erfindung auf ebenso überraschend einfache wie wirkungsvolle Weise durch die erfindungsgemäße Magnetresonanzapparatur wie in Anspruch 1 definiert gelöst.

Erfindungsgemäß wird also eine verbesserte Magnetresonanzapparatur vorgeschlagen, bei welcher eine modifizierte elektrische Schaltung es ermöglicht, einen Strom durch eine Induktivität, vorzugsweise eine Gradientenspule, möglichst schnell auf die Zielstromstärke zu bringen, ohne dass von der elektrischen Schaltung, welche den Gradientenstrom liefert, dauerhaft eine hohe Spannung bereitgestellt werden muss. Hierzu wird eine Hilfsinduktivität bereitgestellt, die mit der Gradientenspule über einen Schalter in Serie geschaltet werden kann. Der Strom lädt bei dieser Schaltung zunächst die Hilfsinduktivität und bei Öffnen des Schalters oder des Transistors baut sich über die Hilfsinduktivität kurzzeitig eine sehr hohe Spannung auf, welche zu einem sehr schnellen Anstiegs des Stroms in der Gradientenspule führt.

Der Lösungsansatz der vorliegenden Erfindung ist im Wesentlichen auf eine verbesserte Steuerschaltung für den Gradientenstrom gerichtet:

In der hier vorgestellten Schaltung wird als Stromlieferant eine niedervoltige Spannungsquelle verwendet. Diese hat, nach der Definition für eine Spannungsquelle, einen sehr kleinen Ausgangswiderstand. Sie liefert also eine nahezu konstante Spannung, unabhängig vom entnommenen Strom. Somit ist es hier möglich eine andere Stromstärke für die Hilfsspule und für die Gradientenspule zu erreichen, ohne dass sich die Spannung der Spannungsquelle merklich ändert. Diese unterschiedlichen Stromstärken werden hier durch einfügen von Widerständen in die Stromwege einerseits nur für die Hilfsspule und andererseits für die Hilfsspule und die Gradientenspule bewirkt. Kann der Strom durch die Hilfsspule so eingestellt werden, dass er größer als der gewünschte Gradientenstrom ist, so kann die Induktivität der Hilfsspule klein gewählt werden. Für gleiche Induktivitätswerte muss der Strom durch die Hilfsspule zweimal dem Gradientenstrom sein, um einen optimal impulsförmigen Stromanstieg durch die Gradientenspule zu erhalten. Wird in der hier beschriebenen Schaltung der Stromfluss durch die Hilfsspule abgeschaltet, so entsteht an ihr eine hohe Spannung. Damit ergibt sich an der Gradientenspule die Summe aus der Versorgungsspannung und der hohen Spannung über der Hilfsspule was zu einem schnellen Anstieg des Gradientenstroms führt.

Um eine Kontrolle über den Strom zu haben, kann eine Regelschaltung benutzt werden, welche die Versorgungsspannung in Abhängigkeit der Stromstärke nachregeln. Auf diese Weise werden Änderungen des Gradientenstroms durch z. B. thermische Effekte im Stromkreis erkannt und korrigiert. Dafür wird der zu messende Strom durch einen Widerstand geleitet, an dem dann eine Spannung entsteht. Diese Spannung wird über einen Differenzverstärker mit einem einstellbaten Sollwert verglichen und die Spannungsquelle entsprechend nachgeregelt. Da hier zeitlich nacheinander unterschiedliche Stromstärken für die Hilfsspule und die Gradientenspule gebraucht werden, es aber vermieden werden soll, dass sich daraus ein Nachregeln der Spannungsquelle ergibt, wird der Strommesswiderstand, abhängig vom benötigten Stromverhältnis aufgeteilt. In der Zeit, wenn der Strom nur durch die Hilfsspule fließt, wird nur ein Teil des Strommesswiderstandes durchflossen, während beim Stromfluss durch die Gradientenspule beide Strommesswiderstände durchflossen werden. Dadurch wird in der hier vorgestellten Schaltung erreicht, dass in beiden Fällen die gleiche Spannung am Eingang des Differenzverstärkers ansteht und somit kein Regelvorgang erfolgt. So wird verhindert, dass sich die Spannung an der Spannungsquelle ändert, wodurch einschwingen des Gradientenstromes vermieden wird.

Bevorzugte Ausführungsformen und Weiterbildungen der Erfindung

Besonders bevorzugt ist eine Ausführungsform der erfindungsgemäßen Magnetresonanzapparatur, bei der die Spannungsquelle so ausgebildet ist, dass sie eine maximale Spannung U zwischen 5V und 30V, vorzugsweise U ≤ 25V, insbesondere U ≈ 15V erzeugen kann. Mit höheren Spannungswerten werden auch die Anforderungen an den Berührungsschutz immer höher.

Weitere vorteilhafte Ausführungsformen sind dadurch gekennzeichnet, dass zumindest ein Teil der Schaltelemente als Transistoren ausgeführt ist. Schnelles Schalten ist so einfach möglich.

Eine weitere vorteilhafte Ausführungsform sieht vor, dass die erste Hilfsspule und die zweite Hilfsspule in Serie mit der Gradientenspule und je nach Stromrichtung in einer wechselseitigen Alternativschaltung gegeneinander angeordnet sind. Die Hilfsspule liefert für kurze Zeit die Spannung, die für den schnellen Stromanstieg nötig ist.

Eine bevorzugte Ausführungsform ist dadurch gekennzeichnet, dass die Induktivität der Hilfsspulen in einem Bereich zwischen 10μH und 10mH, vorzugsweise bei etwa 100μH. liegt. Durch die Möglichkeit des erhöhten Stromflusses durch die Hilfsspule kann deren Induktivitätswert verhältnismäßig klein sein.

Vorteilhaft ist auch eine Ausführungsform, bei der eine Regelschaltung vorhanden ist, die es ermöglicht, einen gewünschten Stromfluss durch die Gradientenspule und/oder die Hilfsspulen einzustellen. Veränderungen, zum Beispiel durch thermische Effekte, können so ausgeglichen werden.

Eine Klasse von besonders bevorzugten Ausführungsformen der erfindungsgemäßen Magnetresonanzapparatur zeichnet sich dadurch aus, dass in der elektrischen Schaltung Strommesswiderstände sowie ein Differenzverstärker vorhanden sind, und dass die niedervoltige Spannungsquelle steuerbar ist, so dass der Betrag des fließenden Stroms durch die Gradientenspule in einer Regelschleife kontrolliert werden kann.

Vorteilhafte Weiterbildungen dieser Klasse von Ausführungsformen sind dadurch gekennzeichnet, dass mittels der Regelschleife die Stromstärke auf dem gewünschten Wert, insbesondere konstant, gehalten werden kann. Auf diese Weise ist zu jedem Zeitpunkt gewährleistet, dass die richtige Stromstärke vorhanden ist.

Alternativ oder ergänzend sind bei anderen vorteilhaften Weiterbildungen in der elektrischen Schaltung die Widerstände so optimiert sind, dass sich im Einschaltmoment des Gradientenstromes nur eine minimale Spannungsänderung an der niedervoltigen Spannungsquelle ergibt. Dadurch wird bewirkt, dass es nur minimales Einschwingen der Spannung gibt und so eine nahezu perfekte Impulsstrom erzeugt werden kann.

Eine weitere vorteilhafte Ausführungsform der erfindungsgemäßen Magnetresonanzapparatur zeichnet sich dadurch aus, dass in der elektrischen Schaltung vor dem ersten Schaltelement der erste elektrischer Widerstand sowie vor weiteren Schaltelementen jeweils ein elektrischer Widerstand vorhanden ist, und dass aufgrund einer Wahl der Widerstandsverhältnisse R1_a / R2_a und R1_b / R2_b der zeitliche Verlauf des Stromanstiegs durch die Gradientenspule beeinflusst wird. Auf diese Weise ist es möglich, auch von der rechteckigen Impulsform abweichende Stromanstiege einzustellen.

In den Rahmen der vorliegenden Erfindung fällt auch ein Verfahren zum Betrieb der elektrischen Schaltung zur Erzeugung variabler, impulsförmiger Ströme durch die Gradientenanordnung in einer Magnetresonanzapparatur gemäß der oben beschriebenen erfindungsgemäßen Ausführungsformen oder ihrer Weiterbildungen, welches sich dadurch auszeichnet,

dass zunächst vor dem Beginn eines gewünschten Gradientenstromflusses das dritte Schaltelement und das erste Schaltelement leitend geschaltet werden, dass nach Erreichen der gewünschten Stromstärke durch die erste Hilfsspule und dem gewünschten Stromfluss durch die Gradientenspule das zweite Schaltelement leitend und das erste Schaltelement nichtleitend geschaltet werden, dass für die entgegengesetzte Stromrichtung das vierte Schaltelement) und das fünfte Schaltelement leitend geschaltet werden,

und dass nach Erreichen der gewünschten Stromstärke durch die zweite Hilfsspule und dem gewünschten Stromfluss durch die Gradientenspule das sechste Schaltelement leitend und das fünfte Schaltelement nichtleitend geschaltet werden.

Weitere Vorteile der Erfindung ergeben sich aus der Beschreibung und der Zeichnung. Ebenso können die vorstehend genannten und die noch weiter ausgeführten Merkmale erfindungsgemäß jeweils einzeln für sich oder zu mehreren in beliebigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung.

Detaillierte Beschreibung der Erfindung und Zeichnung

Die Erfindung ist in den Figuren und Diagrammen der Zeichnung dargestellt und wird anhand von Ausführungsbeispielen näher erläutert.

Es zeigen:

Fig.1 eine schematische Darstellung einer elektrischen Schaltung gemäß einer ersten einfacheren Ausführungsform der Erfindung;

Fig.2 eine schematische Darstellung einer elektrischen Schaltung gemäß einer zweiten Ausführungsform mit Differenzverstärker;

Fig.3 ein schematisches Diagramm der zeitlichen Abfolge der Ansteuerpulse für den genereller Stromfluss 3A, den Stromfluss 3B durch die Hilfsspule und den Stromfluss 3C durch die Gradientenspule; und

Fig.4 ein Verlaufsschema der Verfahrensschritte beim Betrieb der elektrischen Schaltung zur Erzeugung variabler, impulsförmiger Ströme durch die Gradientenanordnung in einer erfindungsgemäßen Magnetresonanzapparatur sowie mit den resultierenden Strömen durch die Hilfsspule und die Gradientenspule für unterschiedliche Stromverhältnisse.

Die Erfindung geht aus von einer Magnetresonanzapparatur mit einem Hauptfeldmagneten zur Erzeugung eines homogenen Magnetfelds in einem Messvolumen, mit einer Gradientenanordnung zum Erzeugen von magnetischen Feldgradienten im Messvolumen, welche mindestens eine **Gradientenspule G** mit einem **Innenwiderstand Rg** umfasst, sowie mit einer elektrischen Schaltung zur Erzeugung variabler, impulsförmiger Ströme durch die Gradientenanordnung, welche ein **erstes Schaltelement Sx1_a** in Serie mit einem **ersten**

**Widerstand R1_a** umfasst, mit dem ein Strom nur durch eine **erste Hilfsspule Ls_a** ein- oder ausgeschaltet werden kann, und wobei die elektrische Schaltung ein **zweites Schaltelement S2_a** umfasst, mit welchem ein serieller Stromfluss durch die Gradientenspule G und die erste Hilfsspule Ls_a geschaltet werden kann.

Diese elektrische Schaltung ist erfindungsgemäß dadurch gekennzeichnet, dass sie mindestens eine **niedervoltige Spannungsquelle V** zur Erzeugung einer Spannung $U \leq 100V$ umfasst, mit der ein definierter Spannungsabfall über dem Innenwiderstand Rg der Gradientenspule G eingestellt werden kann, wobei die erste Hilfsspule Ls_a in der elektrischen Schaltung zwischen der Spannungsquelle V und der Gradientenspule G in Serie mit der Gradientenspule G angeordnet ist, dass in der elektrischen Schaltung ein erster Schaltungsteil vorhanden ist, der ein **drittes Schaltelement S1_a** zwischen der Spannungsquelle V und der ersten Hilfsspule Ls_a umfasst, und dass ein zweiter Schaltungsteil vorhanden ist mit einem **vierten Schaltelement S1_b**, einer **zweiten Hilfsspule Ls_b,** einem **fünften Schaltelement Sx1_b** in Serie mit einem **dritten Widerstand R1_b** und mit einem **sechsten Schaltelement S2_b** in Serie mit einem **vierten Widerstand R2_b,** welcher es ermöglicht, die zum ersten Schaltungsteil entgegengesetzte Stromrichtung in der Gradientenspule G zu erzeugen.

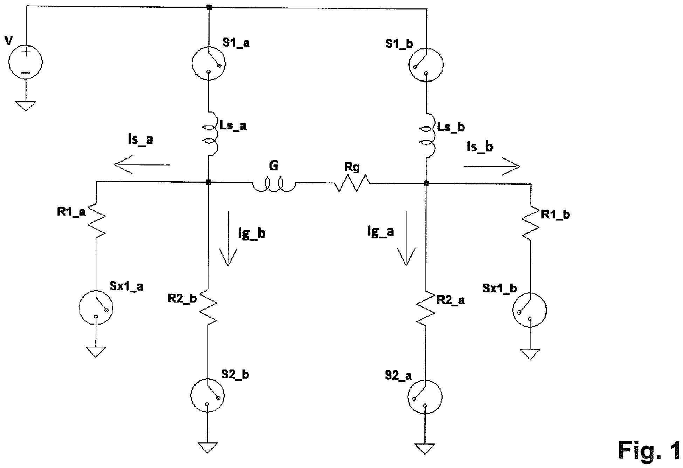

In der Schaltung von **Fig. 1** ist eine einfachere Form der Erfindung realisiert. Die durch die niederohmige Spannungsquelle V erzeugte Spannung liegt dauerhaft an der Schaltung an. Die Spannung U dieser Spannungsquelle V bestimmt den letztendlich fließenden Gradientenstrom Ig = U / R

Hier ist R = Rg + R2_a oder R = Rg + R2_b je nach Polarität des gewünschten Gradientenstromes.

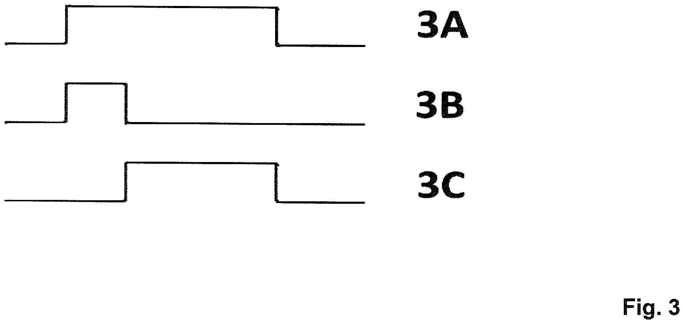

Mit einer steuerbaren Spannungsquelle kann also die Höhe des Gradientenstroms gesteuert werden. Kurze Zeit, bevor der Stromimpuls durch die Gradientenspule G benötigt wird, werden die Schaltelemente S1_a, Sx1_a oder alternativ, je nach gewünschter Stromrichtung des Gradientenstromimpulses, S1_b, Sx1_b leitend geschaltet. Dies ist in Fig. 3 mit 3A für S1_a oder alternativ S1_b und mit 3B für das Schaltelement Sx1_a oder alternativ Sx1_b gezeigt.

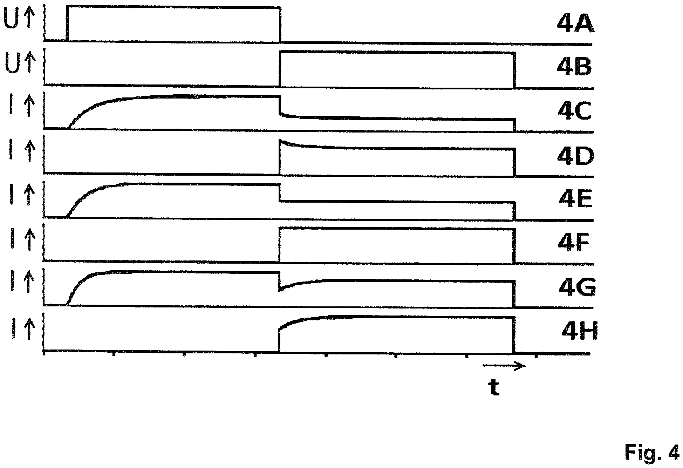

Nach kurzer Zeit, die unter anderem von der Induktivität der Hilfsspule Ls_a oder alternativ Ls_b bestimmt wird, stellt sich ein konstanter Stromfluss durch die Hilfsspule Ls_a oder alternativ Ls_b ein, der unter anderem durch den Widerstand R1_a oder alternativ R1_b und der Ausgansspannung der Spannungsquelle V bestimmt wird. Das wird in Fig. 4 mit den Kurven 4C, 4E und 4G gezeigt.

Wird der Stromimpuls in der Gradientenspule G benötigt, so wird das Schaltelement S2_a oder alternativ S2_b leitend geschaltet und gleichzeitig Sx1_a oder alternativ Sx1_b nichtleitend geschaltet. Das wird in Fig. 3 mit 3B und 3C durch die sich ändernden Pegel der Ansteuerspannung dargestellt.

In Fig. 4, Abschnitt 4A ist der zeitliche Verlauf dieser Schaltvorgänge für Sx1_a oder alternativ Sx1_b und in Abschnitt 4B für das Schaltelement S2_a oder alternativ S2_b gezeigt. Durch das Schalten auf "nichtleitend" entsteht an der Hilfsspule Ls_a oder alternativ Ls_b und somit auch an der Gradientenspule G eine kurzzeitige Spannungsspitze die dafür sorgt, dass der Strom durch die Gradientenspule G extrem schnell ansteigt. Wurde das Verhältnis der Induktivitäten Ls der Hilfsspule Ls_a oder alternativ Ls_b zur Induktivität Lg der Gradientenspule G günstig gewählt, zum Beispiel Ls = Lg, so kann durch die Wahl der Widerstandes R1_a oder alternativ R1_b der Strom durch die Hilfsspule Ls_a oder alternativ Ls_b in der Zeit vor dem Gradientenstromimpuls so eingestellt werden, dass dieser in diesem Beispiel dem doppelten Wert des Gradientenstromimpulses entspricht. Mit dieser Kombination erhält man die bestmögliche Annäherung an eine rechteckige Impulsform des Gradientenstroms. Für diese Gradientenstromimpulsform gilt allgemein:

$$Is / Ig = (Ls + Lg) / Ls$$

Dabei ist Is der Strom durch eine Hilfsspule mit der Induktivität Ls und Ig der gewünschte Gradientenstrom.

Mit von der Gleichung abweichenden Werten, sind die in Diagramm gemäß Fig. 4 gezeigten Formen der Impulsströme möglich. In Fig. 4 zeigen die Abschnitte 4C, 4E und 4G den Stromverlauf durch die Hilfsspule für unterschiedliche Einstellungen der Ströme ls_a oder alternativ ls_b. Dabei zeigt Fig. 4, Abschnitt 4C einen größeren Strom, Fig. 4 Abschnitt 4E den optimale Strom und Fig. 4 Abschnitt 4G einen kleineren Strom als zum Erzeugen eines rechteckigen Stromimpulses nötig ist. Die Kurven Fig.4, Abschnitte 4D, 4F und 4H zeigen die daraus resultierenden Verläufe der Gradientenströme.

Die Widerstände R2_a und R2_b sind nötig, wenn der Innenwiderstand der Gradientenspule so klein ist, dass die Spannung der Spannungsquelle V beim gewünschten Gradientenstrom sehr klein wird. Diese Spannung sollte möglichst >5V sein um eine gute Funktion der Schaltung zu gewährleisten.

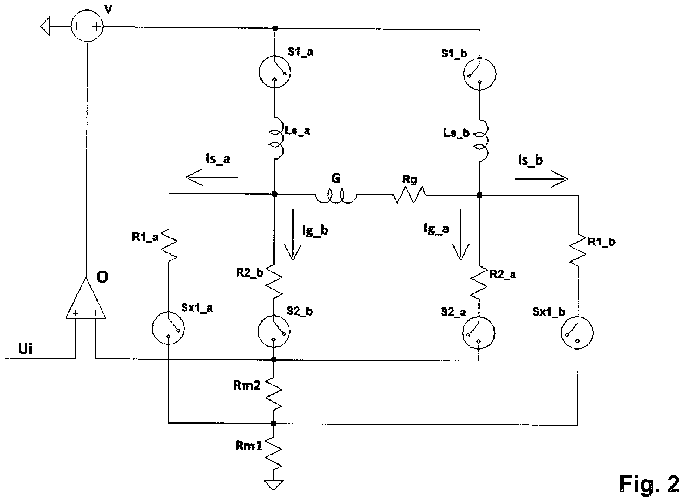

In Schaltung von **Fig. 2** ist eine Form der Erfindung realisiert, bei der die Spannungsquelle V in Abhängigkeit des tatsächlich fließenden Gradientenstromes geregelt wird.

Hier wird die Spannungsquelle durch einen Differenzverstärker O geregelt. Dieser vergleicht die Spannung Ui, mit welcher die Höhe des Gradientenstromes eingestellt werden kann, mit der Spannung an den **Strommess-Widerständen Rm1 und Rm2** und versucht die Spannungsquelle V so einzustellen, dass Gleichheit zwischen den Spannungen besteht.

Wenn bei dieser Schaltung alle Schaltelemente ausgeschaltet sind, also kein Strom fließt, wird die Spannung der Spannungsquelle V durch den Differenzverstärker O auf maximale Spannung eingestellt. Sobald aber für die kurze Zeit vor dem Gradientenstromimpuls der Strom durch die Hilfsspule Ls_a oder alternativ Ls_b und die Schaltelemente S1_a, Sx1_a oder alternativ S1_b, Sx1_b fließt, wird der Stromregelkreis aktiv und die Ausgangsspannung der Spannungsquelle V wird so eingestellt, dass an dem Widerstand Rm1 die gleiche Spannung entsteht, wie sie durch Ui vorgegeben wird.

Der Vorgang des Einregelns des Stromes braucht etwas Zeit, welche aber innerhalb der Ladezeit der Hilfsspule Ls_a oder alternativ Ls_b liegen kann. Die Art, wie sich der Stromfluss aufbaut ist vollkommen unkritisch, weil nur gewährleistet sein muss, dass im Umschaltmoment der benötigte Ladestrom durch eine der Hilfsspulen Ls_a oder alternativ Ls_b fließt.

Das Einschalten des Gradientenstroms geht wie bei Schaltung 1 beschrieben, vor sich. Nach dem Umschalten fließt der Gradientenstrom durch Rm1 und Rm2, wodurch daran eine Spannung entsteht, die dem Differenzverstärker O zugeführt wird. Dieser stellt die Spannungsquelle V so ein, dass Gleichheit mit Ui besteht und somit der gewünschte Gradientenstrom fließt. Um im Umschaltmoment kein Nachregeln des Regelkreises zu verursachen, sollten die Strommess-Widerstände Rm1 und Rm2 so gewählt werden, dass sich am Eingang des Differenzverstärkers O keine Spannungsänderung ergibt. So wird vermieden, dass Zeit zum neu Einregeln des Stromes gebraucht wird und der Gradientenstrom Ig sich in dieser Zeit verändert. Dies ist bei dem Beispiel dann der Fall, wenn die Widerstandswerte von Rm1 und Rm2 gleich sind. Abhängig, unter anderen von Is_a, Is_b, dem Gradientenstrom Ig, Ls_a, Ls_b und der Induktivität der Gradientenspule G mit Lg, sind aber auch andere Widerstandsverhältnisse möglich.

Unter Berücksichtigung aller Innenwiderstände im Stromflussweg für ls_a, ls_b und Ig, wie unter Anderem der Schaltelemente, der Hilfsspulen, der Gradientenspule G und der Spannungsquelle V und den Widerständen Rm1 und Rm2, können die Widerstände R1_a, R2_a, R1_b und R2_b so gewählt werden, dass es trotz einer eventuellen kleinen Spannungsänderung der Spannungsquelle V in Umschaltmoment, keinen Einfluss auf das Verhältnis Is_a / Ig oder alternativ ls_b / Ig gibt. Dies sorgt auch für eine Verbesserung der Impulsform des Gradientenstroms Ig.

Die erfindungsgemäße Schaltung besteht also im Prinzip aus der Serienschaltung einer niedervoltigen Spannungsquelle, einer Hilfs- und einer Hauptinduktivität und einer Regelschaltung, mit der es möglich ist, den gewünschten Strom durch die Induktivitäten einzustellen. Der Strom lädt bei dieser Schaltung zunächst die Hilfsinduktivität und bei Öffnen der Regelschaltung (Transistors) baut sich über die Hilfsinduktivität kurzzeitig eine sehr hohe Spannung auf, welche zu einem sehr schnellen Anstiegs des Stroms in der Hauptinduktivität führt.

Bei vielen Schaltungen nach Stand der Technik ist ein wesentlicher Nachteil, dass hohe Spannungen verwendet werden. Bei anderen Schaltungen nach Stand der Technik wird eine Stromquelle als Stromlieferant für den Gradientenstrom benutzt, was immer zu einem Stromregelvorgang am Beginn des Gradientenstromflusses führt. Mit der beschriebenen erfindungsgemäßen Schaltung kann ein schneller und steilflankiger Gradientenstromimpuls auch schon mit kleinen Betriebsspannungen von z.B. 5V erzeugt werden.

In einer Hilfsinduktivität wird die Energie gespeichert, die zum gewünschten Zeitpunkt für einen sehr schnellen Anstieg des Stromes in der Hauptinduktivität gebraucht wird, ohne dass dafür eine Hochspannungsversorgung benötigt wird. Die Variation der Stromstärke der Impulse ist mit nur einem Stromregelkreis realisiert.

Typische Werte für die Hauptinduktivität in der Verwendung als Gradientenspule in MR Systemen können zwischen wenigen 10 $\mu$H bis hin zum mH-Bereich liegen. Die Schaltung kann vorzugsweise bei Spannungen zwischen 5 - 30V und für Stromstärken von wenigen mA bis zu z.B. 100A benutzt werden, wobei nur die technischen Daten der verwendeten Bauteile die Limits bilden.

In **Fig. 3** ist beispielhaft ein Zeitschema der Ansteuerimpulse für die Schaltelemente gezeigt, in welchem das oben beschriebene erfindungsgemäße Verfahren durchgeführt wird:

3A: Strom ein: S1_a oder alternativ S1_b werden dazu verwendet die Stromflussrichtung durch die Gradientenspule zu bestimmen.

3B: Hilfsspule laden: Sx1_a oder alternativ Sx1_b ermöglichen im leitenden Zustand, dass ein Ladestrom durch eine Hilfsspule Ls_a oder alternativ Ls_b fließt.

3c: Gradientenstrom ein: S2_a oder alternativ S2_b ermöglichen im leitenden Zustand, dass Strom durch die Gradientenspule fließt.

**Fig. 4** schließlich stellt ein zeitliches Verlaufsschema der Verfahrensschritte beim Betrieb der elektrischen Schaltung zur Erzeugung variabler, impulsförmiger Ströme durch die Gradientenanordnung in einer erfindungsgemäßen Magnetresonanzapparatur dar:

4A Ansteuerspannung von Sx1_a oder alternativ Sx1_b

4B Ansteuerspannung von S2_a oder alternativ S2_b

4C Stromverlauf in der Hilfsspule Ls_a oder alternativ Ls_b, wenn der Strom größer ist, als es für einen rechteckigen Gradientenstromanstieg nötig ist

4D Stromverlauf in der Gradientenspule, wenn der Ladestrom größer ist, als es für einen rechteckigen Gradientenstromanstieg nötig ist

4E Stromverlauf in der Hilfsspule Ls_a oder alternativ Ls_b, wenn der Strom so groß ist, wie es für einen rechteckigen Gradientenstromanstieg nötig ist

4F Stromverlauf in der Gradientenspule, wenn der Ladestrom so groß ist, wie es für einen rechteckigen Gradientenstromanstieg nötig ist

4G Stromverlauf in der Hilfsspule Ls_a oder alternativ Ls_b, wenn der Strom kleiner ist, als es für einen rechteckigen Gradientenstromanstieg nötig ist

4H Stromverlauf in der Gradientenspule, wenn der Ladestrom kleiner ist, als es für einen rechteckigen Gradientenstromanstieg nötig ist

Das erfindungsgemäße Verfahren dient dem Betrieb der erfindungsgemäßen elektrischen Schaltung zur Erzeugung variabler, impulsförmiger Ströme durch die Gradientenanordnung in einer entsprechend modifizierten Magnetresonanzapparatur und zeichnet sich dadurch aus,

dass zunächst vor dem Beginn eines gewünschten Gradientenstromflusses das dritte Schaltelement S1_a und das erste Schaltelement Sx1_a leitend geschaltet werden,
dass nach Erreichen der gewünschten Stromstärke Is_a durch die erste Hilfsspule Ls_a und dem gewünschten Stromfluss durch die Gradientenspule G das zweite Schaltelement S2_a leitend und das erste Schaltelement Sx1_a nichtleitend geschaltet werden,
dass für die entgegengesetzte Stromrichtung das vierte Schaltelement S1_b und das fünfte Schaltelement Sx1_b leitend geschaltet werden,
und dass nach Erreichen der gewünschten Stromstärke Is_b durch die zweite Hilfsspule Ls_b und dem gewünschten Stromfluss durch die Gradientenspule G das sechste Schaltelement S2_b leitend und das fünfte Schaltelement Sx1_b nichtleitend geschaltet werden.

**Bezugszeichenliste**

| V | niedervoltige Spannungsquelle |
|---|---|
| Ui | Spannung |
| G | Gradientenspule |
| Ig_a | erster Gradientenstrom |
| Ig_b | zweiter Gradientenstrom |
| Is_a | Strom durch die erste Hilfsspule |
| Is_b | Strom durch die zweite Hilfsspule |
| Rg | Innenwiderstand |
| Sx1_a | erstes Schaltelement |
| S2_a | zweites Schaltelement |
| S1_a | drittes Schaltelement |
| S1_b | viertes Schaltelement |

| | |
|---|---|
| **Sx1_b** | fünftes Schaltelement |
| **S2_b** | sechstes Schaltelement |
| **R1_a** | erster Widerstand |
| **R2_a** | zweiter Widerstand |
| **R1_b** | dritter Widerstand |
| **R2_b** | vierter Widerstand |
| **Rm1** | erster Strommess-Widerstand |
| **Rm2** | zweiter Strommess-Widerstand |
| **Ls_a** | erste Hilfsspule |
| **Ls_b** | zweite Hilfsspule |
| **O** | Differenzverstärker |

**Referenzliste**

Für die Beurteilung der Patentfähigkeit in Betracht gezogene Publikationen:

> [1] DE 33 36 286 A1
> [2] US 2006/0152222 A1
> [3] US-A 4,961,054
> [4] US-A 5,270,657
> [5] US-A 2,941,125
> [6] US-A 5,311,136

**Patentansprüche**

1. Magnetresonanzapparatur mit einem Hauptfeldmagneten zur Erzeugung eines homogenen Magnetfelds in einem Messvolumen, mit einer Gradientenanordnung zum Erzeugen von magnetischen Feldgradienten im Messvolumen, welche mindestens eine Gradientenspule (G) mit Induktivität $Lg$ und einem Innenwiderstand $Rg$ umfasst, sowie mit einer elektrischen Schaltung zur Erzeugung variabler, impulsförmiger Ströme durch die Gradientenanordnung, welche ein erstes Schaltelement (Sx1_a) in Serie mit einem ersten Widerstand (R1_a) umfasst, mit dem ein Strom $Is$ nur durch eine erste Hilfsspule (Ls_a) mit Induktivität $Ls$ ein- oder ausgeschaltet werden kann, und wobei die elektrische Schaltung ein zweites Schaltelement (S2_a) umfasst, mit welchem ein serieller Stromfluss $Ig$ durch die Gradientenspule (G) und die erste Hilfsspule (Ls_a) geschaltet werden kann,
**dadurch gekennzeichnet,**

   **dass** die elektrische Schaltung mindestens eine niedervoltige Spannungsquelle (V) zur Erzeugung einer Spannung $U \le 100V$ umfasst, mit der ein definierter Spannungsabfall über dem Innenwiderstand (Rg) der Gradientenspule (G) eingestellt werden kann, wobei die erste Hilfsspule (Ls_a) in der elektrischen Schaltung zwischen der Spannungsquelle (V) und der Gradientenspule (G) in Serie mit der Gradientenspule (G) angeordnet ist, dass in der elektrischen Schaltung ein erster Schaltungsteil vorhanden ist, der für eine Bestromung der Gradientenspule (G) in einer ersten Richtung ausgelegt ist und das erste Schaltelement (Sx1_a) in Serie mit dem ersten Widerstand (R1_a), ein zweites Schaltelement (S2_a) in Serie mit einem zweiten Widerstand (R2_a) sowie ein drittes Schaltelement (S1_a) zwischen der Spannungsquelle (V) und der ersten Hilfsspule (Ls_a) umfasst, und **dass** ein zweiter Schaltungsteil vorhanden ist mit einem vierten Schaltelement (S1_b) zwischen der Spannungsquelle (V) und einer zweiten Hilfsspule (Ls_b), die zwischen der Spannungsquelle (V) und der Gradientenspule (G) angeordnet ist, mit einem fünften Schaltelement (Sx1_b) in Serie mit einem dritten Widerstand (R1_b) und mit einem sechsten Schaltelement (S2_b) in Serie mit einem vierten Widerstand (R2_b), wobei ein Stromfluss nur durch die zweite Hilfsspule (Ls_b) mittels dem fünften Schaltelement (Sx1_b) oder ein serieller Stromfluss durch die zweite Hilfsspule (Ls_b) und die Gradientenspule (G) in einer zur ersten Richtung entgegengesetzten Richtung mittels dem sechsten Schaltelement (S2_b) schaltbar ist, und wobei gilt: $Is / Ig = (Ls + Lg) / Ls$.

2. Magnetresonanzapparatur nach Anspruch 1, **dadurch gekennzeichnet, dass** die Spannungsquelle (V) so ausgebildet ist, dass sie eine maximale Spannung $U$ zwischen 5V und 30V, vorzugsweise $U \le 25V$, insbesondere $U \approx 15V$ erzeugen kann.

3. Magnetresonanzapparatur nach einem der vorhergehenden Ansprüche, **dadurch gekennzeichnet, dass** zumindest ein Teil der Schaltelemente als Transistoren ausgeführt ist.

4. Magnetresonanzapparatur nach einem der vorhergehenden Ansprüche, **dadurch gekennzeichnet, dass** die Induktivität der Hilfsspulen (Ls_a, Ls_b) in einem Bereich zwischen $10\mu H$ und $10mH$, vorzugsweise bei etwa $100\mu H$. liegt.

5. Magnetresonanzapparatur nach einem der vorhergehenden Ansprüche, **dadurch gekennzeichnet, dass** eine Regelschaltung vorhanden ist, die es ermöglicht, einen gewünschten Stromfluss durch die Gradientenspule (G) und/oder die Hilfsspulen (Ls_a, Ls_b) einzustellen.

6. Magnetresonanzapparatur nach einem der vorhergehenden Ansprüche, **dadurch gekennzeichnet, dass** in der elektrischen Schaltung Strommesswiderstände (Rm1, Rm2) sowie ein Differenzverstärker (O) vorhanden sind, und dass die niedervoltige Spannungsquelle (V) steuerbar ist, so dass der Betrag des fließenden Stroms durch die Gradientenspule (G) in einer Regelschleife kontrolliert werden

**7.** Magnetresonanzapparatur nach Anspruch 6, **dadurch gekennzeichnet, dass** mittels der Regelschleife die Stromstärke auf dem gewünschten Wert, insbesondere konstant, gehalten werden kann.

**8.** Magnetresonanzapparatur nach einem der Ansprüche 6 oder 7, **dadurch gekennzeichnet, dass** in der elektrischen Schaltung die Widerstände (R1_a, R2_a, R1_b, R2_b, Rm1, Rm2) so optimiert sind, dass sich im Einschaltmoment des Gradientenstromes nur eine minimale Spannungsänderung an der niedervoltigen Spannungsquelle (V) ergibt

**9.** Verfahren zum Betrieb der elektrischen Schaltung zur Erzeugung variabler, impulsförmiger Ströme durch die Gradientenanordnung in einer Magnetresonanzapparatur nach einem der vorhergehenden Ansprüche, **dadurch gekennzeichnet,**

**dass** entweder für die Bestromung der Gradientenspule (G) in der ersten Richtung vor dem Beginn des gewünschten Gradientenstromflusses das dritte Schaltelement (S1_a) und das erste Schaltelement (Sx1_a) leitend geschaltet werden,
**dass** nach Erreichen der gewünschten Stromstärke (Is_a) durch die erste Hilfsspule (Ls_a) und dem gewünschten Stromfluss durch die Gradientenspule (G) das zweite Schaltelement (S2_a) leitend und das erste Schaltelement (Sx1_a) nichtleitend geschaltet werden,
oder dass für die Bestromung der Gradientenspule (G) in der entgegengesetzten Stromrichtung das vierte Schaltelement (S1_b) und das fünfte Schaltelement (Sx1_b) leitend geschaltet werden,
und **dass** nach Erreichen der gewünschten Stromstärke (Is_b) durch die zweite Hilfsspule (Ls_b) und dem gewünschten Stromfluss durch die Gradientenspule (G) das sechste Schaltelement (S2_b) leitend und das fünfte Schaltelement (Sx1_b) nichtleitend geschaltet werden.

**Claims**

**1.** Magnetic resonance device having a main field magnet for the generation of a homogeneous magnetic field in a measuring volume, with a gradient arrangement for the generation of magnetic field gradients in said measuring volume, incorporating at least one gradient coil (G) with inductance Lg and an internal resistance Rg, and having an electric circuit for the generation of variable pulse-shaped currents through the gradient arrangement, incorporating a first switching element (Sx1_a) which is series-connected to a first resistor (R1_a), by means of which a current Is can only be switched-on or switched-off in a first auxiliary coil (Ls_a) with inductance Ls, and wherein the electric circuit incorporates a second switching element (S2_a), by means of which a serial current flux Ig can be switched through the gradient coil (G) and the first auxiliary coil (Ls_a), **characterized**

in that the electric circuit comprises at least one low-voltage voltage source (V) for the generation of a voltage U ≤ 100V, by means of which a specific voltage drop across the internal resistance Rg of the gradient coil (G) can be set, wherein the first auxiliary coil (Ls_a) in the electric circuit is arranged between the voltage source (V) and the gradient coil (G), in series with the gradient coil (G),
in that a first circuit section is present in the electric circuit, which is configured to energize the gradient coil (G) in a first direction and comprises the first switching element (Sx1_a) which is series-connected to the first resistor (R1_a), a second switching element (S2_a) which is series-connected to a second resistor (R2_a) and a third switching element (S1_a) between the voltage source (V) and the first auxiliary coil (Ls_a), and in that a second circuit section is present, having a fourth switching element (S1_b) between the voltage source (V) and a second auxiliary coil (Ls_b), which is arranged between the voltage source (V) and the gradient coil (G), and a fifth switching element (Sx1_b) arranged in series with a third resistor (R1_b), and a sixth switching element (S2_b) arranged in series with a fourth resistor (R2_b), wherein a current flow can be switched only through the second auxiliary coil (Ls_b) by means of the fifth switching element (Sx1_b) or a series current flow can be switched through the second auxiliary coil (Ls_b) and the gradient coil (G) in a reverse direction to the first direction by means of the sixth switching element (S2_b), and wherein the following applies: Is / Ig = (Ls + Lg) / Ls.

**2.** Magnetic resonance device according to Claim 1, **characterized in that** the voltage source (V) is configured such that it can generate a maximum voltage U between 5V and 30V, preferably U ≤ 25V, and specifically U ≈ 15V.

**3.** Magnetic resonance device according to one of the preceding claims, **characterized in that** at least some of the switching elements are configured as transistors.

**4.** Magnetic resonance device according to one of the preceding claims, **characterized in that** the inductance of the auxiliary coils (Ls_a, Ls_b) lies within a range of 10µH to 10mH, and is preferably of the order of 100µH.

**5.** Magnetic resonance device according to one of the preceding claims, **characterized in that** a control circuit is present, which permits the setting of a desired current flux in the gradient coil (G) and/or in the auxiliary coils (Ls_a, Ls_b).

**6.** Magnetic resonance device according to one of the preceding claims, **characterized in that**, in the electric circuit, current measuring shunts (Rm1, Rm2) and a difference amplifier (O) are present, and **in that** the low-voltage voltage source (V) is controllable, such that the magnitude of the current flow in the gradient coil (G) can be controlled in a control loop.

**7.** Magnetic resonance device according to Claim 6, **characterized in that**, by means of the control loop, the current strength can be maintained at the desired value, and specifically at a constant value.

**8.** Magnetic resonance device as claimed in one of Claims 6 or 7, **characterized in that** the resistors (R1_a, R2_a, R1_b, R2_b, Rm1, Rm2) in the electric circuit are optimized such that, upon the switch-on of the gradient current, only a minimum voltage variation occurs on the low-voltage voltage source (V).

**9.** Method for operating the electric circuit for the generation of variable pulse-shaped currents in the gradient arrangement of a magnetic resonance device according to one of the preceding claims, **characterized**

   **in that** either for energizing the gradient coil (G) in the first direction prior to the commencement of a desired gradient current flux, the third switching element (S1_a) and the first switching element (Sx1_a) are switched to a conducting state,
   **in that**, further to the achievement of the desired current strength (Is_a) in the first auxiliary coil (Ls_a) and the desired current flux in the gradient coil (G), the second switching element (S2_a) is switched to a conducting state, and the first switching element (Sx1_a) is switched to a non-conducting state,
   or that, for energizing the gradient coil (G) in the reverse current direction, the fourth switching element (S1_b) and the fifth switching element (Sx1_b) are switched to a conducting state,
   and **in that**, further to the achievement of the desired current strength (Is_b) in the second auxiliary coil (Ls_b) and the desired current flux in the gradient coil (G), the sixth switching element (S2_b) is switched to a conducting state, and the fifth switching element (Sx1_b) is switched to a non-conducting state.

**Revendications**

**1.** Appareil de résonance magnétique avec un aimant de champ principal permettant de générer un champ magnétique homogène dans un volume de mesure, avec un agencement de gradient permettant de générer des gradients de champ magnétique dans le volume de mesure, qui comprend au moins une bobine de gradient (G) avec une inductance Lg et une résistance interne Rg, et avec un circuit électrique permettant de générer des courants variables et impulsionnels à travers l'agencement de gradient, qui comprend un premier élément de commutation (Sx1_a) en série avec une première résistance (R1_a), avec lequel un courant Is ne peut être activé ou désactivé que par une première bobine auxiliaire (Ls_a) avec une inductance Ls, ledit circuit électrique comprenant également un deuxième élément de commutation (S2_a) avec lequel un flux de courant sériel Ig peut être commuté à travers la bobine de gradient (G) et la première bobine auxiliaire (Ls_a), **caractérisé en ce que**

   le circuit électrique comprend au moins une source de tension à faible voltage (V) permettant de générer une tension U ≤ 100V, avec laquelle une chute de tension définie peut être ajustée par l'intermédiaire de la résistance interne (Rg) de la bobine de gradient (G), la première bobine auxiliaire (Ls_a) étant agencée dans le circuit électrique en série avec la bobine de gradient (G) entre la source de tension (V) et la bobine de gradient (G),
   **en ce que** dans le circuit électrique se trouve une première partie de circuit conçue pour alimenter la bobine de gradient (G) dans une première direction et comprenant le premier élément de commutation (Sx1_a) en série avec la première résistance (R1_a), un deuxième élément de commutation (S2_a) en série avec une deuxième résistance (R2_a) et un troisième élément de commutation (S1_a) entre la source de tension (V) et la première bobine auxiliaire (Ls_a),
   et **en ce qu'**une deuxième partie de circuit est présente avec un quatrième élément de commutation (S1_b) entre la source de tension (V) et une deuxième bobine auxiliaire (Ls_b) agencée entre la source de tension (V) et la bobine de gradient (G), avec un cinquième élément de commutation (Sx1_b) en série avec une troisième résistance (R1_b) et avec un sixième élément de commutation (S2_b) en série avec une quatrième résistance (R2_b), un flux de courant ne pouvant être commuté qu'à travers la deuxième bobine auxiliaire (Ls_b) au moyen du cinquième élément de commutation (Sx1_b) ou un flux de courant sériel pouvant être commuté à travers la deuxième bobine auxiliaire (Ls_b) et la bobine de gradient (G) dans une direction opposée à la première direction au moyen du sixième élément de commutation (S2_b), et où : $Is / Ig = (Ls + Lg) / Ls$.

2. Appareil de résonance magnétique selon la revendication 1, **caractérisé en ce que** la source de tension (V) est conçue de manière à pouvoir générer une tension maximale U comprise entre 5V et 30V, de manière préférée $U \leq 25V$, de manière particulièrement préférée $U \approx 15V$.

3. Appareil de résonance magnétique selon l'une quelconque des revendications précédentes, **caractérisé en ce qu'**au moins une partie des éléments de commutation sont réalisés sous la forme de transistors.

4. Appareil de résonance magnétique selon l'une quelconque des revendications précédentes, **caractérisé en ce que** l'inductance des bobines auxiliaires (Ls_a, Ls_b) se situe dans une plage comprise entre 10 $\mu$H et 10 mH, de manière préférée est d'environ 100 $\mu$H.

5. Appareil de résonance magnétique selon l'une quelconque des revendications précédentes, **caractérisé en ce qu'**il existe un circuit de régulation qui permet d'ajuster un flux de courant souhaité à travers la bobine de gradient (G) et/ou les bobines auxiliaires (Ls_a, Ls_b).

6. Appareil de résonance magnétique selon l'une quelconque des revendications précédentes, **caractérisé en ce que** des résistances de mesure de courant (Rm1, Rm2) ainsi qu'un amplificateur différentiel (O) sont présents dans le circuit électrique et **en ce que** la source de tension à faible voltage (V) peut être commandée de sorte que la quantité du courant circulant à travers la bobine de gradient (G) peut être contrôlée au sein d'une boucle de régulation.

7. Appareil de résonance magnétique selon la revendication 6, **caractérisé en ce que** l'intensité de courant peut être maintenue à la valeur souhaitée, en particulier constante, au moyen de la boucle de régulation.

8. Appareil de résonance magnétique selon l'une quelconque des revendications 6 ou 7, **caractérisé en ce que** les résistances (R1_a, R2_a, R1_b, R2_b, Rm1, Rm2) sont optimisées au sein du circuit électrique de sorte qu'il n'en résulte qu'une modification minimale de la tension au niveau de la source de tension à faible voltage (V) au moment de l'activation du courant de gradient.

9. Procédé de fonctionnement du circuit électrique permettant de générer des courants variables et impulsionnels grâce à l'agencement de gradient au sein d'un appareil de résonance magnétique selon l'une quelconque des revendications précédentes, **caractérisé en ce que**

soit le troisième élément de commutation (S1_a) et le premier élément de commutation (Sx1_a) sont commutés de manière conductrice en vue de l'alimentation de la bobine de gradient (G) dans la première direction avant le début du flux de courant de gradient souhaité, et après avoir atteint l'intensité de courant (Is_a) souhaitée à travers la première bobine auxiliaire (Ls_a) et le flux de courant souhaité à travers la bobine de gradient (G), le deuxième élément de commutation (52_a) est commuté de manière conductrice et le premier élément de commutation (Sx1_a) est commuté de manière non conductrice,

soit le quatrième élément de commutation (S1_b) et le cinquième élément de commutation (Sx1_b) sont commutés de manière conductrice en vue de l'alimentation de la bobine de gradient (G) dans la direction de courant opposée, et après avoir atteint l'intensité de courant souhaitée (Is_b) à travers la deuxième bobine auxiliaire (Ls_b) et le flux de courant souhaité à travers la bobine de gradient (G), le sixième élément de commutation (S2_b) est commuté de manière conductrice et le cinquième élément de commutation (Sx1_b) est commuté de manière non conductrice.

**Fig. 1**

Fig. 2

3A

3B

3C

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 3336286 A1
- US 20060152222 A1
- US 4961054 A
- US 5270657 A
- US 2941125 A
- US 5311136 A